# EUROPÄISCHE PATENTSCHRIFT

(11) **EP 1 820 518 B1**
(45) Veröffentlichungstag und Bekanntmachung des Hinweises auf die Patenterteilung: **17.09.2008**
(21) Anmeldenummer: 06002921.2
(22) Anmeldetag: 14.02.2006
(51) Int. Cl.: A61L 2/18, A61L 2/26, B67D 5/58, B08B 3/08, C01B 15/01

(54) **Anordnung zum Dosieren von flüssigen Gefahrgütern aus einem Vorratsbehälter**
Arrangement for dosing of dangerous liquids from a storage container
Arrangement de dosage de liquides dangereux à partir d'un récipient de stockage

(43) Veröffentlichungstag der Anmeldung: 22.08.2007
(73) Patentinhaber: Chemische Fabrik Dr. Weigert GmbH & Co. KG, 20539 Hamburg (DE)
(72) Erfinder: Lensch, Jan, 23826 Bark (DE)
(74) Vertreter: Glawe, Delfs, Moll

(56) Entgegenhaltungen:
- EP-A- 0 266 573
- EP-A- 1 121 942
- WO-A-94/07544
- DE-A1- 3 819 419

## Beschreibung

Die Erfindung betrifft eine Anordnung zum Dosieren von flüssigen Gefahrgütern aus einem Vorratsbehälter in ein Nutzsystem.

Im gewerblichen und industriellen Bereich werden häufig flüssige Gefahrgüter eingesetzt. Diese werden am oder in der Nähe des Verwendungsortes bevorratet und dann je nach Bedarf einem Nutzsystem (eine Anordnung oder ein System, in dem die Flüssigkeit Verwendung findet) zudosiert (vergl. EP-A-0266573).

Im Bereich der Krankenhaushygiene, beispielsweise bei der Reinigung, Sterilisierung und/oder Desinfektion medizinischer und chirurgischer Instrumente, werden im Zuge der Aufbereitung der Instrumente unter Umständen Gefahrgüter eingesetzt. In der Lebensmittel- und Getränkeindustrie werden u.a. für die CIP-Reinigung und Desinfektion von Tanks, Rohrleitungen, Wärmetauschern etc. große Mengen Gefahrgüter eingesetzt. Beispielsweise kann als Desinfektionsmittel eine peroxidhaltige Flüssigkeit wie Peressigsäure Verwendung finden. Peressigsäure gast aus und muss daher mit besonderen Vorsichtsmaßnahmen gehandhabt und gelagert werden. Beim Eintrag von Fremdstoffen, insbesondere oxidierbaren Fremdstoffen, kann Peressigsäure sehr heftig reagieren. Aus Vorsichtsgründen können daher solche Flüssigkeiten nur in verhältnismäßig kleinen Gebinden gehandhabt und gelagert werden. Dies ist aufwendig.

Der Erfindung liegt die Aufgabe zugrunde, eine eingangsgenannten Anordnung zu schaffen, die ein gefahrloses und sicheres Dosieren solcher Flüssigkeiten gestattet, so dass die Verwendung größerer und damit wirtschaftlicher Gebinde möglich ist.

Erfindungsgemäß weist die Anordnung folgende Merkmale auf:
a) einen geschlossenen Vorratsbehälter mit einem Fassungsvermögen von wenigstens 100 l,
b) eine Entnahmeleitung zur Entnahme von Flüssigkeit aus dem Vorratsbehälter,
c) einen von der Entnahmeleitung gespeisten Vorlagebehälter, der gleichzeitig als Rückschlagsicherung zur Verhinderung eines Rückflusses von Flüssigkeit in den Vorratsbehälter ausgebildet ist,
d) eine Ansaugleitung, mittels der Flüssigkeit aus dem Vorlagebehälter zu einer Dosierpumpe geführt wird,
e) eine Dosierpumpe zum Fördern der Flüssigkeit,
f) eine Speiseleitung, die die Druckseite der Dosierpumpe mit dem Nutzsystem verbindet,
g) eine Rückflussleitung, die überschüssige Flüssigkeit aus dem Nutzsystem und/oder der Dosierpumpe in den Vorlagebehälter zurückführt.

Zunächst seien einige im Rahmen der Erfindung verwendete Begriffe erläutert. Der Vorratsbehälter ist ein Lagerbehälter für das flüssige Gefahrgut, aus dem die laufende Speisung des Nutzsystems erfolgt. Die Speisung erfolgt im Rahmen der Erfindung über den zwischengeschalteten Vorlagebehälter. Bei einem Nutzsystem kann es sich beispielsweise um eine CIP-Reinigungsanlage für die Lebensmittel- oder Getränkeindustrie oder eine Vorrichtung zur Reinigung, Desinfektion und/oder Sterilisierung medizinischer Instrumente handeln. Der Vorratsbehälter ist geschlossen, dies bedeutet, dass unerwünschte Umwelteinflüsse von der darin gelagerten Flüssigkeit fern gehalten werden. Sein Fassungsvermögen für das flüssige Gefahrgut beträgt wenigstens 100 l.

Die Anordnung weist eine Entnahmeleitung auf, mittels der die Flüssigkeit entnommen und dem Nutzsystem zugeführt wird. Es kann sich beispielsweise um eine Entnahmelanze handeln, die durch eine an der Oberseite des Behälters angeordnete Öffnung bis in dessen Bodenbereich geführt wird.

Diese Entnahmeleitung speist einen Vorlagebehälter. In diesem Vorlagebehälter wird eine bestimmte Menge Flüssigkeit vorgelegt, die dann weiter dem Nutzsystem zugeführt wird. Dieser zwischen Vorratsbehälter und Nutzsystem geschaltete Vorlagebehälter ist gleichzeitig als Rückschlagsicherung zur Verhinderung eines Rückflusses von Flüssigkeit in den Vorratsbehälter ausgebildet. Wie oben bereits erwähnt, können Gefahrgüter wie Peressigsäure beim Eintrag von Verunreinigungen heftig reagieren. Im Betrieb einer Reinigungsanordnung oder Spülmaschine für medizinische Instrumente kann es jedoch insbesondere bei Störungen zu einem Rückschlag von Flüssigkeit in Richtung des Vorratsbehälters kommen. Beispielsweise können Zufuhrschläuche verstopft oder abgeknickt sein, so dass Flüssigkeit vom Saugeingang einer Dosierpumpe zurückschlägt. Bei bestimmten Anlagen können auch Bypässe vorgesehen sein, durch die überschüssige von einer Pumpe geförderte Flüssigkeit in Richtung des Vorratsbehälters zurücktritt. Solche zurückschlagende Flüssigkeit kann Verunreinigungen enthalten. Erfindungsgemäß ist daher der Vorlagebehälter vorgesehen, in dem solche rückschlagende Flüssigkeit aufgefangen werden kann.

Ein weiterer Vorteil des Vorlagebehälters ist, dass er einen Kurzzeitvorrat von Flüssigkeit speichern kann. Zu diesem Zweck ist das Fassungsvermögen des Vorlagebehälters vorzugsweise so ausgebildet, dass ihm die für einen Betriebszyklus des Nutzsystems (beispielsweise einen Reinigungs/Desinfektionszyklus einer Spülmaschine) erforderliche Flüssigkeitsmenge enthalten sein kann. Läuft während des Spülbetriebs der Vorratsbehälter leer, kann mittels des im Vorlagebehälter gespeicherten Flüssigkeitsvorrats der entsprechende Betriebszyklus noch zu Ende geführt und muss nicht unterbrochen werden.

Aus dem Vorlagebehälter wird die Flüssigkeit mittels einer Ansaugleitung zu einer Dosierpumpe geführt. Die Dosierpumpe fördert die Flüssigkeit in einer vorgegebenen Menge zum Nutzsystem. Die Flüssigkeit kann vor der Einspeisung in das Nutzsystem mit anderen Flüssigkeiten vermischt oder beispielsweise mit Wasser verdünnt werden. Die Druckseite der Dosierpumpe ist mit dem Nutzsystem mittels einer Speiseleitung verbunden. Wenn es bei bestimmten Betriebszuständen oder Störungen im Nutzsystem zu einem Rückschlag von Flüssigkeit kommt, ist erfindungsgemäß ferner eine Rückflussleitung vorgesehen, die überschüssige Flüssigkeit aus der Dosierpumpe in den Vorlagebehälter zurückführt. Die dort zwischengespeicherte überschüssige Flüssigkeit wird dort im weiteren oder aber im nächsten Betriebszyklus des Nutzsystems abgefördert und verbraucht. Ein Zurückschlagen bis in den großvolumigen Vorratsbehälter ist ausgeschlossen. Die Rückflussleitung kann insbesondere mit der Dosierpumpe verbunden sein, so dass bei einem übergroßen Gegendruck aus Richtung des Nutzsystems (beispiels-weise durch eine Störung) druckseitig anfallende überschüssige Flüssigkeit in den Vorlagebehälter zurückgeführt wird.

Das flüssige Gefahrgut ist bevorzugt eine ausgasende Flüssigkeit, insbesondere eine peroxidhaltige Flüssigkeit. Das Nutzsystem ist bevorzugt eine CIP-Reinigungsanlage für die Lebensmittel- oder Getränkeindustrie oder eine Einrichtung zum maschinellen Reinigen und/oder Desinfizieren von medizinischen und/oder chirurgischen Instrumenten und/oder Apparaten.

Der Vorratsbehälter weist bevorzugt ein Fassungsvermögen von wenigstens 200 1, weiter vorzugsweise wenigstens 500 l, weiter vorzugsweise wenigstens 1.000 l auf. Beispielsweise kann ein Großgebinde mit 1.000 l Fassungsvermögen verwendet werden. Die Entnahmeleitung weist bevorzugt ein in den Vorratsbehälter ragende Entnahmelanze auf. Die Entnahmeleitung kann als weitere Sicherung gegen Rückschlag eine separate, zusätzliche Rückflusssicherung aufweisen. Dabei kann es sich beispielsweise um ein Rückschlagventil handeln.

Ein bevorzugtes Fassungsvermögen des Vorlagebehälters liegt zwischen 5 und 20 1, bevorzugt bei etwa 10 l. Das Fassungsvermögen ist dasjenige Volumen, das der Vorlagebehälter sicher aufnehmen kann, ohne dass es zu einem Rückschlag in den Vorratsbehälter kommen kann.

Bevorzugt ist es, wenn die Entnahmeleitung, die Flüssigkeit aus dem Vorratsbehälter zuführt, im Vorlagebehälter oberhalb des Flüssigkeitsspiegels mündet. Die Ansaugleitung befindet sich bevorzugt im unteren Bereich des Vorlagebehälters unterhalb des Flüssigkeitsspiegels. Durch dies Anordnung von Entnahme und Ansaugleitung fungiert der Vorlagebehälter automatisch als Rückschlagsicherung.

Bevorzugt weist der Vorlagebehälter eine Einrichtung zum Abführen von aus der Flüssigkeit austretendem Gas auf. Es kann sich hier beispielsweise um ein Überdruckventil handeln. Sofern Verunreinigungen aus dem Nutzsystem in den Vorlagebehälter zurückschlagen, kann es zu einem starken Ausgasen der peroxidhaltigen Flüssigkeit kommen. Da der Vorlagebehälter nur ein verhältnismäßig geringes Flüssigkeitsvolumen enthält, bleibt diese Reaktion kontrollierbar und das entstehende Gas kann über das Überdruckventil abgeführt werden.

Als zusätzliche Sicherung kann der Vorlagebehälter eine Einrichtung zum Überwachen des Flüssigkeitsniveaus aufweisen. Es kann vorgesehen sein, dass beim Unterschreiten eines Mindestflüssigkeitsniveaus oder beim Überschreiten eines Maximalflüssigkeitsniveaus eine automatische Abschaltung der Dosierpumpe erfolgt. Alternativ und/oder zusätzlich kann ein Alarm ausgelöst werden. Durch diese Sicherheitseinrichtungen wird verhindert, dass die Dosierpumpe trocken läuft oder dass bei außergewöhnlichen Betriebsstörungen der Vorlagebehälter so voll wird, dass doch noch ein Rückschlagen in den Vorratsbehälter droht.

Es kann vorgesehen sein, dass auch beim Erreichen des Maximalflüssigkeitsniveaus noch ein erheblicher Gasraum im Vorlagebehälter oberhalb des Flüssigkeitsspiegels vorgesehen ist. Dies ist eine zusätzlich Sicherheitsmaßnahme, um auch bei diesem Flüssigkeitsniveau ein etwaiges Ausgasen unter Kontrolle zu halten. Dieser Gasraum oberhalb des maximalen Flüssigkeitsspiegels kann beispielsweise in etwa dem Fassungsvermögen des Vorlagebehälters für Flüssigkeit entsprechen.

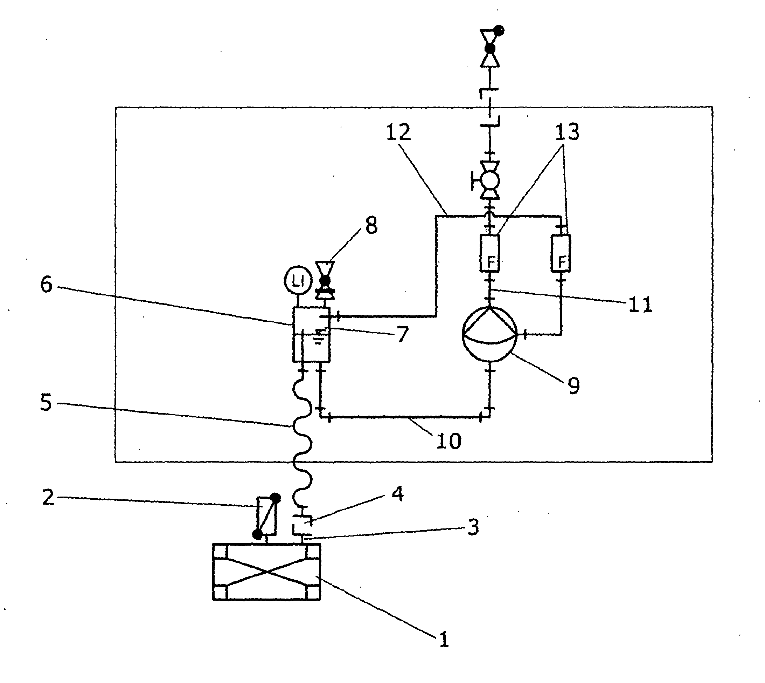

Ein Ausführungsbeispiel der Erfindung wird im Folgenden anhand der Zeichnung erläutert.

Ein Container 1 für Peressigsäure weist ein Fassungsvermögen von 1.000 l auf. Ein Ventil 2 ist für eine Belüftung des Containers im Zuge der Entnahme der Flüssigkeit vorgesehen. Das Belüftungsventil 2 kann zusätzlich als Überdruckventil zum Ableiten austretenden Gases ausgebildet sein, alternativ kann zusätzlich ein solches Ventil vorgesehen sein. Eine bei 3 angedeutete Entnahmelanze ragt etwa bis zum Boden des Containers. Mittels einer bei 4 angedeuteten Flüssigkeitskupplung wird eine Entnahmeleitung 5 angeschlossen. Diese führt in einen Vorlagebehälter 6 aus PVC, der ein Fassungsvermögen für Flüssigkeit von 10 l aufweist. In dem in der Zeichnung gezeigten Zustand ist das Maximalflüssigkeitsniveau in diesem Vorlagebehälter erreicht. Man erkennt, dass die Entnahmeleitung 5 oberhalb dieses Maximalflüssigkeitsniveaus mündet und damit einen Rückschlag von Flüssigkeit in den Container 1 verhindert. Ein bei 7 angedeuteter Schwimmerschalter überwacht das Flüssigkeitsniveau im Vorlagebehälter 6 und gibt beim Überschreiten des Maximalflüssigkeitsniveaus ein Alarmsignal bzw. schaltet die Dosierpumpe 9 ab. Der Vorlagebehälter 6 ist unterdruckfest. Ein Überdruckventil 8 kann beim Ausgasen der Flüssigkeit im Vorlagebehälter 6 entstehenden Überdruck entweichen lassen.

Im unteren Bereich des Vorlagebehälters 6 mündet eine Ansaugleitung 10, die mit dem Sauganschluss der Dosierpumpen 9 verbunden ist. Eine Speiseleitung 11 führt vom Druckanschluss der Dosierpumpe 9 in Richtung des Nutzsystems.

Eine Rückschlagleitung 12 führt von der Druckseite Dosierpumpe 9 zurück zum Vorlagebehälter 6. Speiseleitung 11 und Rückflussleitung 12 sind mit Durchflusssensoren 13 versehen.

Die Dosierpumpe 9 weist im Betrieb eine konstante Förderleistung auf. Sie saugt Flüssigkeit aus dem Vorlagebehälter 6 und fördert sie in die Speiseleitung 11. Durch das Absinken des Flüssigkeitsspiegels im Vorlagebehälter 6 entsteht darin ein Unterdruck, der dazu führt, dass über die Entnahmeleitung 5 Flüssigkeit aus dem Vorratsbehälter 1 nachläuft.

Im Betrieb kann es zu Zuständen kommen, denen das Nutzsystem nicht die gesamte Förderleistung der Dosierpumpe 9 abverlangt. Beispielsweise können Schläuche im Nutzsystem (oder auch die Speiseleitung 11) verstopft oder abgeknickt sein oder aber Nutzsystem weist zusätzliche Absperr- oder Dosierventile auf, die zeitweilig die Zufuhr von Flüssigkeit aus der Speiseleitung 11 unterbinden. In diesem Fall wird die Pumpe über den Sensor 13 abgeschaltet.

Von der Dosierpumpe 9 kann in solchen Fällen Flüssigkeit zurückschlagen. Da ein solcher Flüssigkeitsrückschlag nur bis in den Vorlagebehälter 6 stattfinden kann, kann es allenfalls zu kleinen und beherrschbaren Ausgasungsreaktionen kommen.

## Patentansprüche

1. Anordnung zum Dosieren von flüssigen Gefahrgütern aus einem Vorratsbehälter in ein Nutzsystem, wobei die Anordnung folgende Merkmale aufweist:
a) einen geschlossenen Vorratsbehälter (1) mit einem Fassungsvermögen von wenigstens 100 1,
b) eine Entnahmeleitung (5) zur Entnahme von Flüssigkeit aus dem Vorratsbehälter (1),
c) einen von der Entnahmeleitung (5) gespeisten Vorlagebehälter (6), der gleichzeitig als Rückschlagsicherung zur Verhinderung eines Rückflusses von Flüssigkeit in den Vorratsbehälter (1) ausgebildet ist,
d) eine Ansaugleitung (10), mittels der Flüssigkeit aus dem Vorlagebehälter (6) zu einer Dosierpumpe (9) geführt wird,
e) eine Dosierpumpe (9) zum Fördern der Flüssigkeit,
f) eine Speiseleitung (11), die die Druckseite der Dosierpumpe (9) mit dem Nutzsystem verbindet,
g) eine Rückflussleitung (12), die überschüssige Flüssigkeit aus dem Nutzsystem und/oder der Dosierpumpe (9) in den Vorlagebehälter (6) zurückführt.

2. Anordnung nach Anspruch 1, **dadurch gekennzeichnet, dass** der Vorratsbehälter (1) ein Fassungsvermögen von wenigstens 200 1, vorzugsweise wenigstens 500 1, weiter vorzugsweise wenigstens 1.000 1 aufweist.

3. Anordnung nach einem der Ansprüche 1 bis 2, **dadurch gekennzeichnet, dass** die Entnahmeleitung (5) eine in den Vorratsbehälter ragende Entnahmelanze (3) aufweist.

4. Anordnung nach einem der Ansprüche 1 bis 3, **dadurch gekennzeichnet, dass** die Entnahmeleitung (5) eine zusätzliche Rückflusssicherung aufweist.

5. Anordnung nach einem der Ansprüche 1 bis 4, **dadurch gekennzeichnet, dass** der Vorlagebehälter (6) ein Fassungsvermögen von 5 - 20 1, vorzugsweise etwa 10 1 aufweist.

6. Anordnung nach einem der Ansprüche 1 bis 5, **dadurch gekennzeichnet, dass** die Entnahmeleitung (5) im Vorlagebehälter (6) oberhalb des Flüssigkeitsspiegels mündet.

7. Anordnung nach einem der Ansprüche 1 bis 6, **dadurch gekennzeichnet, dass** die Ansaugleitung (10) im unteren Bereich des Vorlagebehälters (6) mündet.

8. Anordnung nach einem der Ansprüche 1 bis 7, **dadurch gekennzeichnet, dass** der Vorlagebehälter (6) eine Einrichtung (8) zum Abführen von aus der Flüssigkeit austretendem Gas aufweist.

9. Anordnung nach Anspruch 8, **dadurch gekennzeichnet, dass** die Einrichtung ein Überdruckventil (8) aufweist.

10. Anordnung nach einem der Ansprüche 1 bis 9, **dadurch gekennzeichnet, dass** der Vorlagebehälter (6) eine Einrichtung (7) zum Überwachen des Flüssigkeitsniveaus aufweist.

11. Anordnung nach Anspruch 10, **dadurch gekennzeichnet, dass** die Einrichtung (7) zum Abschalten der Dosierpumpe (9) bei Unterschreiten eines Mindestflüssigkeitsniveaus im Vorlagebehälter (6) ausgebildet ist.

12. Anordnung nach Anspruch 10 oder 11, **dadurch gekennzeichnet, dass** die Einrichtung (7) zum Abschalten der Dosierpumpe (9) bei Überschreiten eines Mindestflüssigkeitsniveaus im Vorlagebehälter (6) ausgebildet ist.

## Claims

1. Arrangement for dosing dangerous liquids from a storage container into a useful system, with the arrangement having the following features:
a) a closed storage container (1) having a storage capacity of at least 100 1,
b) an extraction line (5) for extracting liquid out of the storage container (1),
c) a reservoir (6) which is fed from the extraction line (5) and which is at the same time designed as a return-flow safeguard for preventing a return flow of liquid into the storage container (1),
d) a suction line (10), by means of which liquid is conducted out of the reservoir (6) to a dosing pump (9),
e) a dosing pump (9) for feeding the liquid,
f) a feed line (11) which connects the pressure side of the dosing pump (9) to the useful system,
g) a return flow line (12) which returns excess liquid from the useful system and/or from the dosing pump (9) into the reservoir (6).

2. Arrangement according to Claim 1, **characterized in that** the storage container (1) has a storage capacity of at least 200 1, preferably at least 500 1, more preferably at least 1000 1.

3. Arrangement according to one of Claims 1 to 2, **characterized in that** the extraction line (5) has an extraction lance (3) which projects into the storage container.

4. Arrangement according to one of Claims 1 to 3, **characterized in that** the extraction line (5) has an additional return-flow safeguard.

5. Arrangement according to one of Claims 1 to 4, **characterized in that** the reservoir (6) has a storage capacity of 5 - 20 l, preferably approximately 10 l.

6. Arrangement according to one of Claims 1 to 5, **characterized in that** the extraction line (5) opens out in the reservoir (6) above the liquid level.

7. Arrangement according to one of Claims 1 to 6, **characterized in that** the suction line (10) opens out in the lower region of the reservoir (6).

8. Arrangement according to one of Claims 1 to 7, **characterized in that** the reservoir (6) has a device (8) for discharging gas which is released from the liquid.

9. Arrangement according to Claim 8, **characterized in that** the device has an overpressure valve (8).

10. Arrangement according to one of Claims 1 to 9, **characterized in that** the reservoir (6) has a device (7) for monitoring the liquid level.

11. Arrangement according to Claim 10, **characterized in that** the device (7) is designed to shut off the dosing pump (9) in the event of a minimum liquid level in the reservoir (6) being undershot.

12. Arrangement according to Claim 10 or 11, **characterized in that** the device (7) is designed to shut off the dosing pump (9) in the event of a minimum liquid level in the reservoir (6) being exceeded.

## Revendications

1. Dispositif de dosage de matières dangereuses liquides à partir d'un réservoir de stockage dans un système utile, dans lequel le dispositif présente les caractéristiques suivantes :
a) un réservoir de stockage fermé (1) avec une contenance d'au moins 100 L,
b) une conduite de prélèvement (5) pour prélever du liquide dans le réservoir de stockage (1),
c) un collecteur (6) alimenté par la conduite de prélèvement (5), lequel sert en même temps de système anti-retour pour empêcher un reflux de liquide dans le réservoir de stockage (1),
d) une conduite d'aspiration (10) au moyen de laquelle le liquide est transporté du collecteur (6) à une pompe de dosage (9),
e) une pompe de dosage (9) pour acheminer le liquide,
f) une conduite d'alimentation (11) qui relie le côté de refoulement de la pompe de dosage (9) au système utile,
g) une conduite de reflux (12) qui renvoie le liquide en excès depuis système utile et/ou la pompe de dosage (9) vers le collecteur (6).

2. Dispositif selon la revendication 1, **caractérisé en ce que** le réservoir de stockage (1) présente une contenance d'au moins 200 L, de préférence d'au moins 500 L, de manière tout particulièrement préférée d'au moins 1 000 L.

3. Dispositif selon l'une quelconque des revendications 1 à 2, **caractérisé en ce que** la conduite de prélèvement (5) présente une lance de prélèvement (3) qui rentre dans le réservoir de stockage.

4. Dispositif selon l'une quelconque des revendications 1 à 3, **caractérisé en ce que** la conduite de prélèvement (5) présente un système anti-retour supplémentaire.

5. Dispositif selon l'une quelconque des revendications 1 à 4, **caractérisé en ce que** le collecteur (6) présente une contenance de 5 à 20 L, de préférence d'environ 10 L.

6. Dispositif selon l'une quelconque des revendications 1 à 5, **caractérisé en ce que** la conduite de prélèvement (5) débouche dans le collecteur (6) au-dessus du niveau de liquide.

7. Dispositif selon l'une quelconque des revendications 1 à 6, **caractérisé en ce que** la conduite d'aspiration (10) débouche dans la zone inférieure du collecteur (6).

8. Dispositif selon l'une quelconque des revendications 1 à 7, **caractérisé en ce que** le collecteur (6) présente un dispositif (8) servant à évacuer le gaz dégagé par le liquide.

9. Dispositif selon la revendication 8, **caractérisé en ce que** le dispositif présente une soupape de surpression (8).

10. Dispositif selon l'une quelconque des revendications 1 à 9, **caractérisé en ce que** le collecteur (6) présente un dispositif (7) servant à surveiller le niveau de liquide.

11. Dispositif selon la revendication 10, **caractérisé en ce que** le dispositif (7) est conçu pour arrêter la pompe de dosage (9) si le niveau de liquide est en-deçà d'un niveau de liquide minimal dans le collecteur (6).

12. Dispositif selon la revendication 10 ou 11, **caractérisé en ce que** le dispositif (7) est conçu pour arrêter la pompe de dosage (9) si le niveau de liquide est au-delà d'un niveau de liquide minimal dans le collecteur (6).
